# EUROPEAN PATENT SPECIFICATION

(11) **EP 4 023 260 B1**
(45) Date of publication and mention of the grant of the patent: **22.04.2026**
(21) Application number: 21216941.1
(22) Date of filing: 22.12.2021
(51) Int. Cl.: A61L 15/22, A61L 15/42, A61L 15/44, A61L 15/46

(54) **NON-WOVEN FABRIC FOR CLEANING, DEBRIDING, REACTIVATING AND/OR DRESSING WOUNDS**
VLIESSTOFF ZUM REINIGEN, DEBRIDIEREN, REAKTIVIEREN UND/ODER VERBINDEN VON WUNDEN
TISSU NON TISSÉ POUR NETTOYER, DÉBRIDER, RÉACTIVER ET/OU PANSER LES BLESSURES

(30) Priority: 30.12.2020 IT 202000032732
(43) Date of publication of application: 06.07.2022
(73) Proprietor: Welcare Industries S.p.A. Società Benefit, 20121 Milano MI (IT)
(72) Inventor: Lazzarotto, Fulvia, 05020 Montecchio TR (IT)
(74) Representative: Di Giovine, Paolo

(56) References cited:
- US-A1- 2020 345 890
- US-B2- 8 030 231

## Description

The present invention relates to a non-woven fabric for cleansing, debriding, reactivating and/or dressing wounds, in particular non-healing wounds, comprising or consisting of viscose fibers, polyethylene terephthalate fibers (PET) and polypropylene fibers (PP), as well as a mitten or glove comprising or consisting of said non-woven fabric as defined in the appended set of claims. The present invention also relates to a non-woven fabric soaked in an aqueous solution for the treatment of wounds as defined in the appended set of claims.

### STATE OF PRIOR ART

In the last few years, the scientific tests have increased proving that the biofilm represents a key pathology in the non-healing lesions. An adequate biofilm management is then vital to reduce the progressive development thereof and to lead the lesion to a definite healing. The concept of *Wound Hygiene* was introduced at the beginning of 2019 during a meeting of an International advisory committee of experts. The committee came to the conclusion that almost all non-healing lesions include a biofilm which delays or prevents their healing itself. The consent document published on vol. 29, Nr. 3 of March 2020 of Journal of Wound Care recommends to treat the non-healing lesions through an early anti-biofilm strategy, called *"Wound Hygiene"* which is divided into 4 steps: cleansing, debriding, reactivating and dressing.

In the current state, the biofilm management involves a regular lesion debriding followed by strategies preventing the re-formation thereof, including the use of topical antimicrobial dressing. This consent document instead suggests the need for going further and implementing a new strategy based upon the lesion hygiene which provides two additional steps: cleansing the lesion and the perilesional skin and reactivating the lesion edges/margins.

The main principle of the *Wound Hygiene* is to remove or reduce to the minimum all unwished materials (including biofilm, devitalized tissues and foreign bodies). This immediately creates the conditions conducive to healing. Like each hygiene form, the effectiveness of *Wound Hygiene* lies in the repetition: the lesion has to be cleansed, debrided and its edges/margins reactivated upon each evaluation and dressing change. Just like hygiene in general, it is not an optional activity.

The new recommendations for the treatment of non-healing wounds have been then so synthesized, through the 4 steps of *Wound Hygiene:*
1) cleansing the lesion bed to remove the devitalized tissue, debris and biofilm. However, rinsing with water and saline solutions is not sufficient, solutions and/or suitable tools are required to prepare effectively the lesion bed for the subsequent debriding step. This procedure has to be performed with the maximum physical force that the patient can tolerate, moreover the committee encourages the use of antiseptics containing surfactants or solutions having balanced pH to cleanse both the lesion bed and the perilesional skin, whereas the highly cytotoxic solutions, such as those containing iodopovidone and hydrogen peroxide, are not recommended.
2) Debriding, that is removing, the necrotic tissue, debris and the residual biofilm upon each dressing change. Debriding can be of different types (mechanical, surgical, ultrasound, biological or a combination thereof). The selection of the debriding method has to be based upon the evaluation of the lesion bed, the perilesional skin and the pain levels and tolerance of the patient. The mechanical force, in combination with a surfactant or an antimicrobial solution, are effective ways to break down and remove the biofilm. The combined use of a cleansing solution for lesions based upon topical surfactants and a debriding buffer or gauze will improve cleansing sufficiently as to break down and remove the biofilm. Debriding "reclaims" the lesion bed and removes biofilm, in this way by preparing it for the dressing application.
3) Reactivating: removing from the lesion edges the necrotic, crusty and/or projecting tissue which can still house the biofilm. Guaranteeing that the skin margins are levelled with the lesion bed to ease the epithelial advancing and the contraction of the lesion itself.
4) Dressing: managing the residual biofilm, by using dressing containing antibiofilm and/or antimicrobial agents to prevent or delay the re-formation thereof.

In such scenario therefore the need is still much felt for having available tools or products suitable and effective for cleansing, debriding, reactivating and dressing non-healing wounds which are in accordance to the principles dictated by *Wound Hygiene.* US 2020/345890 A1 discloses wound dressings that can comprise a non-woven material which is saturated with a pain relieving composition. The dressing debrides the wound.

### SUMMARY OF THE INVENTION

The authors of the present invention have developed a medical device product of class II for cleansing, debriding, reactivating and/or dressing wounds, in particular non-healing wounds, meeting the recommendations of the consent document published on vol. 29, Nr. 3 of March 2020 of Journal of Wound Care*.*

The invention in particular consists in designing a non-woven fabric for cleansing, debriding, reactivating and/or dressing wounds, comprising or consisting of viscose fibers, polypropylene fibers (PP) and polyethylene terephthalate fibers (PET) as defined in the appended set of claims.

The non-woven fabric, the present invention relates to, is characterized by an optimum performance in cleansing, debriding, reactivating and/or dressing wounds which is not only the result of the particular selection of fiber composition, but even of the technique for implementing the same, by "needlepunch" technology. Said non-woven fabric is so defined since it is not implemented by weaving or knitting and it does not require to transform fibers into yarn. According to an aspect of the present invention, said non-woven fabric is characterized by fibers tied therebetween mechanically so as to form multiple both needle and ring structures *("loop technology"),* which result to be much more delicate in the wound cleaning process than the "needle point" structures existing in other products. These fibers advantageously allow to keep the devitalized tissue, debris and biofilm, by removing them from the wound bed.

In an embodiment, the invention combines the mechanical action of the exclusive and new fabric, with the action of an aqueous solution for the treatment of wounds, according to a preferred embodiment a composition particularly effective for cleansing and debriding wounds will be used, thus meeting all requirements of *"Wound Hygiene".*

The soaking solution has all features requested by the recent recommendations for a correct treatment of wounds, in particular non-healing wounds. It is a solution based upon Poloxamer, a surfactant which breaks the surface tension on the wound bed at the interface between a liquid and a solid and eases the removal of the devitalized tissue. Allantoin, another component, is a light keratolytic agent useful in softening and cleansing the dead cells of skin and which acts on hyperkeratosis on the perilesional area. Aloe vera, at last, provides an optimum hydration level to improve the skin integrity, it has anti-inflammatory and antimicrobial properties.

All these features make that the product meets the recommendations included in the consent document published on Journal of Wound Care, vol. 29, Nr.3 of March 2020 and included references.

Advantageously, the fibers used to implement the non-woven fabric, the invention relates to, confer the same unique mechanical features, by guaranteeing compactness and elasticity apart from a correct impregnation with the solution.

According to an aspect of the invention, the fabric can even be welded by ultrasound technique, thus allowing the formation of a glove/mitten which can be soaked in any one of the previously described solutions. The so-obtained medical device allows the operator the hand full mobility while debriding different types of wounds. The *"Wound Hygiene",* in fact, recommends by the operator the maximum physical force that the patent can tolerate and the maximum precision on the wound edges. Both sides of the invention glove result to be humidified with the functional ingredients of the solution, thus allowing the operator to turn the glove and to proceed with cleaning the perilesional area and the surrounding skin.

Therefore, the present invention relates to:
- a non-woven fabric for cleansing, debriding, reactivating and/or dressing wounds comprising or consisting of viscose fibers, polyethylene terephthalate fibers (PET) and polypropylene fibers (PP) as defined in the appended set of claims;
- a glove or mitten comprising or consisting of a non-woven fabric as defined in the appended set of claims;
- a kit for cleansing, debriding, reactivating and/or dressing wounds comprising a non-woven fabric and/or a glove or mitten as defined in the appended set of claims, and an aqueous solution as defined in the appended set of claims.

It is also herein disclosed, as useful to understand the present invention, a process for the preparation of a non-woven fabric for cleansing, debriding, reactivating and/or dressing wounds, comprising the following steps:
i. arranging viscose fibers, polypropylene fibers and polyethylene terephthalate fibers;
ii. homogenizing said fibers;
iii. carding said fibers so as to form plural uniform plies of said fibers;
iv. positioning said plies of fibers obtained in point iii. on each other;
v. mechanically joining said plies of fibers by "needlepunch" technology;
vi. subjecting the non-woven fabric obtained with the previous step to a thermal fixing process.

Additional advantages, as well as the features and use modes of the present invention will result evident from the following detailed description of some preferred embodiments.

### DETAILED DESCRIPTION OF FIGURES

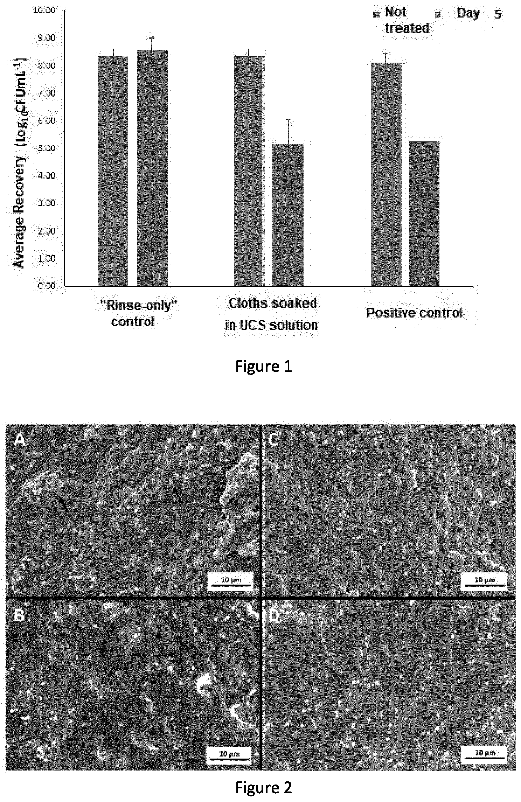

Figure 1. Amount of *Staphylococcus aureus* recovered from biofilms. The blue samples are the not treated ones, the violet samples are ones treated after cleaning processes with: only rinsing (rinse-only, saline solution), UCS cloth and positive controls (sterile gauze + solution of sodium hypochlorite) after 5 days. The error bars represent standard deviations.
Figure 2. Scanning electron micrographs of *Staphylococcus aureus* biofilm formed on the pig skin treated and displayed on the third day. (A) Not treated control; (B) Control only with rinsing; (C) After treatment with UCS system; (D) After treatment with positive control (solution of sodium hypochlorite and sterile gauze). The arrows show one single bacterium. Magnification = 2000 X. Scale = 10 µm.

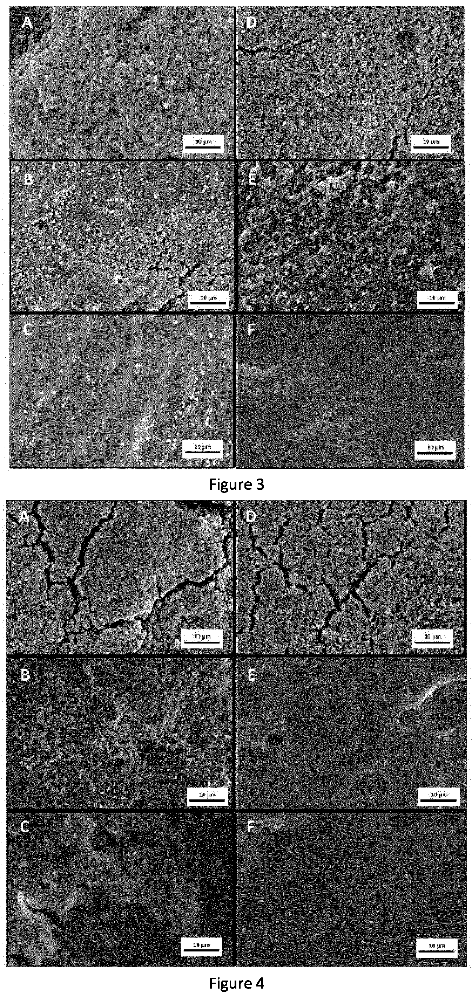

Figure 3. Scanning electron micrographs of *Staphylococcus aureus* biofilm formed on pig skin treated and displayed on the fourth day. (A) Control only with rinsing (before the second rinsing); (B) cloth soaked in UCS solution before the second cleaning; (C) Positive control before the second cleaning; (D) Control only with rinsing (after the second rinsing); (E) after the second cleaning with cloth soaked in UCS solution; (C) after second cleaning with positive control; Magnification = 2000 X. Scale = 10 µm.
Figure 4. Scanning electron micrographs of *Staphylococcus aureus* biofilm formed on pig skin treated and displayed on the fifth day. (A) Control only with rinsing (before the third rinsing); (B) cloth soaked in UCS solution before the third cleaning; (C) Positive control before the third cleaning; (D) Control only with rinsing (after the third rinsing); (E) after the third cleaning with cloth soaked in UCS solution; (C) after the third cleaning with positive control; Magnification = 2000 X. Scale = 10 µm.

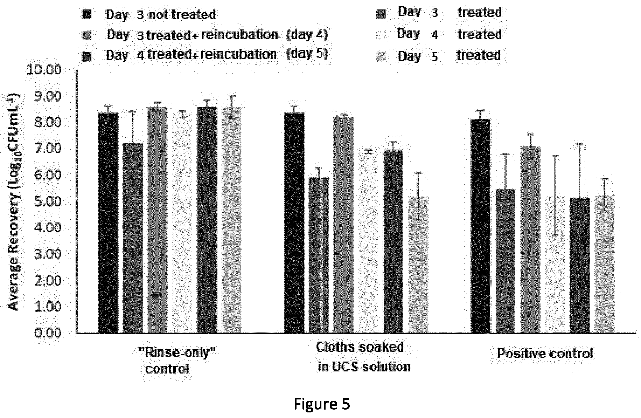

Figure 5. Amount of Staphylococcus aureus recovered from (not treated) preformed 72-hour biofilms and after a series of treatment and re-incubation steps. The error bars represent the standard deviations.

### DETAILED DESCRIPTION OF THE INVENTION

### GLOSSARY

The terms used in the present description are as generally understood by the person skilled in the art, except where differently designated.

Under the term "biofilm", in the present description, a complex community of bacteria and/or fungi of different species is meant, causing a chronic subclinical infection of the wounds.

Where specified, the unit of measurement "decitex", abbreviated "dtex", represents the unit of measurement for the linear density used for determining the title (or titration) of the fibers, corresponding to 1 gram on 10 kilometres.

In the context of the present description, the terms "soaked", "impregnated" and/or "imbibed" are used as synonyms.

As mentioned above, the authors of the present invention have devised a process for implementing a medical device of class II, a non-woven fabric for cleansing, debriding, reactivating and/or dressing wounds meeting in a surprisingly effective way all requirements provided in the consent document published on the vol. 29, Nr. 3 of March 2020 of Journal of Wound Care in the field of Wound Hygiene*.*

A first aspect of the present invention then relates to a non-woven fabric for cleansing, debriding, reactivating and/or dressing wounds comprising or consisting of viscose fibers, polypropylene fibers (PP) and polyethylene terephthalate fibers (PET), wherein said viscose fibers are present in an amount comprised between 40 and 70%, said PET fibers are present in an amount comprised between 15 and 30%, and said polypropylene fibers are present in an amount comprised between 15 and 30% with respect to the total weight of the fabric.

According to an aspect of the present invention, the fibers comprising or consisting of said non-woven fabric, are mechanically tied together so as to form multiple needle and ring structures. Under needle structures the usual structures characterizing a non-woven fabric obtained by needlepunch technology are meant; the ring structures are obtained with partially re-wound fibers or fibers not processed as needlepunch. The advantages of not having only needle structures inside the non-woven fabric consist in making the debriding action more delicate and less painful for the patient.

According to an aspect of the present invention, said fibers made of viscose represent the majoritarian component of the non-woven fabric. The majoritarian component made of viscose is decisive for the wettability and to guarantee a high ease of impregnation of the non-woven fabric, apart from obtaining an optimum delicacy in debriding the wounds, an aspect characterizing this invention. The minority components made of PET and PP, thermoplastic resins, apart from guaranteeing the ultrasound weldability of the non-woven fabric to form, for example, a glove or a mitten, advantageously confer to the non-woven fabric optimum physical-mechanical properties for cleansing, debriding, reactivating and/or dressing wounds, as shown in Table 1 of the present description.

A preferred embodiment according to the present invention in particular relates to a non-woven fabric for cleansing, debriding, reactivating and/or dressing wounds, wherein said viscose fibers are present in an amount equal to 55%, said PET fibers are present in an amount equal to 25%, and said polypropylene fibers are present in an amount equal to 20% with respect to the total weight of the non-woven fabric.

Fibers suitable for the implementation of a non-woven fabric according to the present invention are in particular viscose fibers, PET fibers and staple PP fibers.

Viscose fibers, PET fibers and polypropylene fibers suitable to be used for the implementation of a non-woven fabric according to the present invention are staple fibres having a linear density comprised between 0.5 and 7 dtex and a length (or cut) comprised between 10 and 120 mm. According to an aspect of the invention, said viscose fibers and said PP fibers have a linear density equal to 1.7 dtex and a length comprised between 30 and 50 mm, whereas said PET fibers have a linear density equal to 3.3 dtex and a length comprised between 40 and 80 mm.

According to an aspect of the invention, said viscose fibers and said polypropylene fibers have a linear density equal to 1.7 dtex and a length equal to 40 mm, and said polyethylene terephthalate fibers have a linear density equal to 3.3 dtex and a length equal to 60 mm.

The non-woven fabric according to any one of the previously described embodiments has a weight comprised between 77 and 143 g/m², preferably equal to 110 g/m², and/or a thickness comprised between 1 and 1.62 mm, preferably equal to 1.33 mm.

According to an aspect of the invention, said non-woven fabric is characterized by a percentage elongation at break in orthogonal direction (CD) comprised between 37-102%, and/or a tensile strength in orthogonal direction (CD) comprised between 50-128 N/5cm. In the present description under "orthogonal direction" (CD) the orthogonal direction with respect to the direction in which the machine fabric flows is meant.

As it is known to a person skilled in the art, the above-mentioned mechanical properties can be determined by subjecting a non-woven fabric of the invention to a series of mechanical tests according to the standard methods known in the art. Examples of standard methods which can be used to determine the mechanical properties of the non-woven fabric include, for example, the tests mentioned in column 2 of Table 1 of the present description.

Advantageously, the non-woven fabric according to any one of the herein described embodiments has an adsorption capacity comprised between 976-1496%, measured according to ISO 9073-6.

A preferred embodiment according to the invention in particular relates to a non-woven fabric according to any one of the previously described variants, all having the physical-mechanical features as shown in table 1 of the present description.

As previously mentioned, the herein described specific composition of the non-woven fabric allows the weldability thereof by ultrasound technique. The fabric welding, obtained by ultrasound, allows to form a glove/mitten which guarantees to the operator to have the hand full mobility during debriding of different types of wounds. Therefore, an aspect of the present invention relates to a non-woven fabric according to any one of the previously described embodiments in form of glove or mitten, in particular wherein said non-woven fabric is welded by ultrasound technique so as to form said glove or mitten.

Advantageously, thanks to its high wettability and ease of impregnation, the non-woven fabric according to any one of the herein described embodiments can be soaked in a solution suitable for the treatment of wounds, in particular an aqueous solution for cleansing, debriding, reactivating and/or dressing wounds.

Not limiting examples of solutions suitable for cleansing, debriding, reactivating and/or dressing wounds include for example solutions comprising surfactants and/or antiseptic agents such as polyhexamethylene, octenidine dihydrochloride, hypochlorous acid, or chlorhexidine gluconate.

According to an aspect of the present invention, said non-woven fabric is soaked in an aqueous solution comprising an extract of *Aloe barbadensis* (or aloe vera), Allantoin and/or poloxamer.

Said aqueous solution can also include linolealamidopropyl pg-dimonium chloride phosphate, disodium EDTA, sodium benzoate, polysorbate 20 and/or benzalkonium chloride.

A preferred embodiment of the present invention in particular relates to a non-woven fabric according to any one of the previously described variants soaked in an aqueous solution consisting by 91.82% of water, 5.80% of poloxamer 188, 0.2% of allantoin, 0.2% of disodium EDTA, 0.15% of sodium benzoate, 0.03% of extract of leaves of aloe barbadensis, 0.6% of polysorbate 20, 1% of linolealamidopropyl pg-dimonium chloride phosphate, and 0.2% of 50% solution of benzalkonium chloride.

According to an aspect of the present invention, the non-woven fabric in any one of the previously described embodiments is sterile. The sterilization process of the non-woven fabric can be performed by using any one of the sterilization techniques known in the art. In a preferred embodiment of the present invention, said non-woven fabric is made sterile by means of a procedure as described in the Italian patent application Nr. 102018000008267.

An additional aspect of the invention relates to a glove or a mitten comprising or consisting of a non-woven fabric according to any one of the previously described variants, as defined in the appended set of claims.

Preferably, said glove or mitten is soaked in a solution suitable for cleansing, debriding, reactivating and/or dressing wounds, for example a solution in any one of the previously described embodiments.

As already highlighted, the non-woven fabric, or the glove or mitten comprising or consisting of said non-woven fabric according to any one of the herein described variants, have optimum features for an effective treatment of the wounds. Said non-woven fabric, and/or glove/mitten including it, can be used first of all in cleansing the wounds, for example to ease the biofilm removal, the devitalized tissue, debris, the slough, the colliquate necroses, and/or the residues of preceding dressing existing in a lesion bed. Such procedure allows to decontaminate the lesioned skin and to prepare it effectively for debriding.

Advantageously, said non-woven fabric and/or glove/mitten including it can be used with particular effectiveness in the mechanical debriding of wounds, that is in the physical removal and/or breaking-down of the necrotic tissue, devitalized tissues, foreign bodies, and/or residual biofilm existing on the wounds, without damaging the perilesional skin. In particular, the combined use of the non-woven fabric of the invention with a suitable imbibing solution as defined previously, is capable of improving the wound cleansing sufficiently so as to break down and remove the biofilm.

The non-woven fabric and/or a glove/mitten including it according to the invention, can even be used in reactivating the edges or margins of the wounds, that is to remove the biofilm, callous tissue, hyperkeratose debris and/or senescent cells from the edges/margins of the lesions, allowing to expose the healthy tissue and to promote the advancing thereof. The reactivating stimulates the expression of growth factors to start the formation of a healthy skin.

The non -woven fabric and/or glove or mitten including it according to the invention can be further used, particularly in combination with a suitable aqueous solution as defined previously, for dressing wounds, that is to manage the residual biofilm on the wounds by preventing and/or delaying the reformation thereof. In this step, said non-woven fabric or glove/mitten including it could be used even in combination with microbial agents or topical antibiofilms commonly used in dressing for wounds; they include, for example, polyhexamethylene biguanide (PHMB), iodopovidone, or silver-ethylendiaminotetracetic acid (EDTA)-benzethonium chloride.

According to an aspect of the present invention, said non-woven fabric or glove or mitten according to any one of the previously described embodiments can be used in particular for cleansing, debriding, reactivating and/or dressing non-healing skin wounds/lesions. Said non-woven fabric or glove or mitten according to the invention are suitable for wounds such as, for example, acute wounds, chronic wounds, bedsores, venous and/or arterial ulcers, ischemic ulcers, diabetic ulcers, 1^{st} and 2^{nd}-degree burns, fistulas and abscesses.

It is herein also disclosed, as useful to understand the present invention, a process for the preparation of a non-woven fabric for cleansing, debriding, reactivating and/or dressing wounds, comprising the following steps:
i. arranging viscose fibers, polypropylene fibers and polyethylene terephthalate fibers;
ii. homogenizing said fibers;
iii. carding said fibers so as to form plural uniform plies of said fibers;
iv. positioning said plies of fibers obtained in point iii. on each other;
v. mechanically joining said plies of fibers by "needlepunch" technology;
vi. subjecting the non-woven fabric obtained with the previous step to a thermal fixing process.

As mentioned previously, viscose fibers, polypropylene fibers and polyethylene terephthalate fibers suitable to be used in a process useful to understand the present invention are staple fibers. In said step i., said staple fibers, for example in form of bales, or rolls, or cardboards including them, can be placed in an area arranged for opening, cleaning and mixing fibers.

The homogenisation step ii. of the process useful to understand the invention can be performed by using devices for opening and/or mixing fibers according to any one of the techniques known in the art. By pure way of example, said staple fibers can be fed in an opener and/or blender device: the opening and mixing machines separate the fibers and mix the fibers of different bales/rolls or cardboards. During this procedure most dirt and impurities which may be present are removed due to the effect of the force of gravity and/or of the centrifugal force.

In a variant of the process useful to understand the invention, said viscose fibers are used in an amount equal to 55%, said PET fibers are used in an amount equal to 25%, and said polypropylene fibers are used in an amount equal to 20% with respect to the total weight of the fibers.

According to a variant useful to understand the present invention, said viscose fibers and said polypropylene fibers have a linear density equal to 1.7 dtex and a length equal to 40 mm, and said polyethylene terephthalate fibers have a linear density equal to 3.3 dtex and a length equal to 60 mm.

In step iii. of the process useful to understand the invention carding machines are used which allow to form uniform plies of said fibers. According to a variant useful to understand the invention, the steps of the process from iii. to v. in particular provide for the use of needles made of steel with fine thorns to tangle the short stable fibers made of coherent fabric; in this way a uniform and homogeneous ply is obtained, formed from the mix of fibers which is reinforced mechanically through needles in vertical motion, and which is further tied mechanically in several steps, by adding other layers until obtaining the wished weight of the non-woven fabric.

The thermal fixing process of the non-woven fabric obtained by means of the process useful to understand the present invention can be performed in oven; in this way it is also possible to confer a three-dimensional orientation to the fibers.

It is herein also disclosed as useful to understand the present invention, a process according to any one of the herein described variants, comprising an additional step:
vii. cutting and welding said non-woven fabric so as to obtain a glove or a mitten.

In said step vii, said non-woven fabric can be welded preferably by ultrasound technology.

The process according to any one of the herein described variants useful to understand the invention can further include the following step: imbibing said non-woven fabric with an aqueous solution suitable for cleansing, debriding, reactivating and/or dressing wounds, preferably with an aqueous solution comprising an Aloe barbadensis extract, allantoin and poloxamer, more preferably with an aqueous solution consisting by 91.82% of water, 5.80% of poloxamer 188, 0.2% of allantoin, 0.2% of disodium EDTA, 0.15% of sodium benzoate, 0.03% of extract of leaves of aloe barbadensis, 0.6% of polysorbate 20,1% of linolealamidopropyl pg-dimonium chloride phosphate, and 0.2% of 50% solution of benzalkonium chloride.

The process, useful to understand the present invention, can include an additional step for sterilizing the non-woven fabric obtained at the end of step vi. and/or the glove or mitten obtained at the end of step vii. Said sterilization step can be performed even before or after the imbibition step of the non-woven fabric and/or of the glove or mitten with the aqueous solution as described previously.

The sterilization can be performed by means of any one of the techniques known to a person skilled in the art. In a variant of the process according to any one of the previously described variants and useful to understand the present invention, said sterilization step is performed by means of a process as described in the Italian patent application Nr. 102018000008267.

It is herein also described as useful to understand the invention a non-woven fabric and/or a glove or mitten which can be obtained by means of a process according to any one of the previously described embodiments.

A process for cleansing, debriding, reactivating and/or dressing wounds, in particular non-healing wounds, is herein also described, comprising a step of treating said wounds with a non-woven fabric and/or a glove or mitten according to any one of the previously described embodiments.

Examples are reported here below, which have the purpose of better illustrating the methodologies detected in the present description, such examples have not to be considered in any way as a limitation of the preceding description and of the following claims.

### EXAMPLES

**Example** 1 - Non-woven fabric consisting of viscose fibers (55% by weight) and polypropylene fibers (20% by weight) having a linear density equal to 1.7 dtex and a length equal to 40 mm, and polyethylene terephthalate fibers (25% by weight) having a linear density equal to 3.3 dtex and a length (or cut) equal to 60 mm, imbibed with an aqueous solution based upon allantoin, poloxamer and aloe barbadensis.

**Table 1: functional requirements and performance of the non-woven fabric**

| **Property** | **Measuring method** | **Unit** | **Target** | **Lower specification limit** | **Upper specification limit** |
|---|---|---|---|---|---|
| Elongation (CD) | DIN EN 29073-3 / ISO | % | 69.00 | 37.00 | 102.00 |
| Tensile strength (CD) | DIN EN 29073-3 / ISO | N/5cm | 89.00 | 50.00 | 128.00 |
| Thickness | DIN EN 29073-2 / ISO | Mm | 1.33 | 1.04 | 1.62 |
| Basis weight | DIN EN 29073-1 / ISO | g/m² | 110.00 | 77.00 | 143.00 |
| Elongation (MD) | DIN EN 29073-3 / ISO | % | 36.00 | 18.00 | 54.00 |
| Tensile strength (MD) | DIN EN 29073-3 / ISO | N/5cm | 101.00 | 44.00 | 158.00 |
| Adsorption capacity | ISO 9073-6 | % | | 976.00 | 1496.00 |
| Spec. Micro | AQC-MH-008-H | In 1 g | | | 0.00 |
| Total aerobic microbes | AQC-MH-008-H | CFU | | | 100.00 |
| Total count of yeasts/mildew | AQC-MH-008-H | CFU | | | 10.00 |

### Example 2 - Preliminary study to evaluate the effectiveness of the non-woven fabric

In 2008 a first preliminary study was performed on a prototype of the non-woven fabric exemplified in Example 1. Such study highlighted the *in vivo* effectiveness of the non-woven fabric in cleaning hands (on not damaged skin). Table 2 shows the logarithmic values calculated (averages of right and left hands) for the value of starting microbe content, that is found after the contamination and before treatment (VI), for the value of the final microbe content, that is after treatment with (VF) and the relative logarithmic reduction factor. From the results the reduction of the bacteria content is clear (logarithmic reduction factor equal to 3.03). The contamination has occurred with known bacteria strains of hands and skin.

**Table 2: Logarithmic values of the initial and final bacteria content, or after treatment with imbibed non-woven fabric, and reduction factor.**

| | ***Log VI*** | ***Log VF*** | ***RF*** |
|---|---|---|---|
| ***Average values*** | *7.58* | *4.55* | *3.03* |

### Example 3 - Effectiveness of the non-woven fabric in biofilm removal

The effectiveness of three repeated treatments with a sterile cloth imbibed with a solution based upon aloe barbadensis extracted from the leaves, allantoin and poloxamer (designated hereinafter as UCS solution) was evaluated in the removal of preformed biofilms (*Staphylococcus aureus* NCTC 8325) and in the prevention of recolonization in 5 days by using a pig skin model.

### Formation of biofilm on ex-vivo pig skin

24-hour cultures of *Staphylococcus aureus* were collected from agar-tryptone-soya (TSA) and used to prepare suspensions having 1x10⁸± 5 x 10⁷ CFUmL⁻¹. The bacterial suspensions were diluted in tryptone soya broth (TSB) to prepare working suspensions having 1x10³± 5 x 10² CFUmL⁻¹. The working suspensions were confirmed by means of serial dilutions and transfer on plates. One hundred microliters of the working solutions were distributed on 2x4-cm sections of *ex-vivo* Gamma-sterilized pig skin (day 0). The inoculated pig explants were incubated for 72 hours at 37 ± 2°C under humidified conditions to form the biofilm.

### Treatment of pre-formed biofilms with cloth imbibed with the UCS solution

After 72 hours from incubation (Day 3), three washing steps with PBS (phosphate buffered saline) were performed on each sample so as to remove the plankton bacteria before treatment. A set of samples was immediately recovered after washing (N=3) and a sample was fixed for analysis by scanning electron microscopy (SEM) according to the local procedures. The immediately recovered samples represent the not treated controls. The remaining samples were treated with cloths imbibed with UCS solution, rinsed (rinse-only control) or treated with the positive control as follows:
- a set of samples was treated with cloth imbibed with UCS solution. The treatment with an imbibed cloth was performed by cleaning the pig skin clockwise for 30 seconds. The skin was cleaned again counter-clockwise for further 30 seconds.
- A set of samples was treated with PBS only (rinse-only control). The treatment was performed by cleansing the pig skin with 7 mL of PBS so as to mime the wound irrigation.
- A set of samples was treated with a gauze previously dipped into 7 mL of a bleach-based product (positive control). The treatment was performed by cleaning the pig skin clockwise for 30 seconds. The skin the was cleaned again counter-clockwise for further 30 seconds.

After the treatment, all samples were washed three times in 20 mL of PBS to remove plankton bacteria. A set of samples was introduced in 20 mL of "quench" solution and the remaining living organisms were recovered by sonication (N=3). The "quench" solution includes tween 80 (30 g), lecithin (30 g), histidine (1g), sodium thiosulfate (4g), buffer at pH 7 (20 mL) and distilled water (1 L). The resulting suspension was subjected to serial dilutions, transferred on agar plates and incubated overnight at 37 ± 2°C. The number of colonies was counted and expressed as CFUmL⁻¹, recovery Log₁₀CFUmL⁻¹ and reductions Log₁₀CFUmL⁻¹. A sample was fixed according to the local procedures for analysis by SEM. The sets of remaining samples were incubated for further 24 hours at 37 ± 2°C so as to allow a recolonization of the microorganisms and the biofilm reformation. These processes were repeated two more times on day 4 and on day 5.

### Display of biofilms treated and not treated by means of scanning electron microscopy

The samples were fixed in 2.5% glutaraldehyde for 24 hours. After fixation, the samples were dehydrated by immersion in growing concentrations of ethanol for 10 minutes per concentration (30%, 50%, 70%, 90%, 95% and 100%). The samples were analysed by using a scanning electron microscope JEOL JSM-6010LV.

### Statistical analysis

A Student's t-Test (2-code, two samples same variance) was performed on the samples. The data were considered significant for a value of p < 0.05.

### Results

In this study it was possible to display, thanks to the electron scanning microscopy, the not treated and treated biofilms, by evaluating both the removal thereof after three repeated uses with the cloth imbibed with UCS solution, and after two repeated cleaning. The comparison was performed with an antimicrobial solution based upon sodium hypochlorite on sterile gauze sterile ("*positive control*") and with a saline solution with phosphate buffer ("*rinse-only control*") which simulates the solutions generally used for debriding the wounds with those products on the market appearing as cloths or dry mittens. In many cases the competition products in fact suggest the use of such saline solution for debriding.

In this study the wound debriding was performed after 72 hours of biofilm formation. Debriding was continued for further two days to imitate the clinical use. The results, shown in figure 1, confirm that three treatments repeated with the fabric soaked in UCS solutions led to a decrease up to 3 logarithmic units of *Staphylococcus aureus,* with a significant decrease in the biofilm formation over time before and after each cleaning. A result comparable with that observed by using the antimicrobial agent based upon sodium hypochlorite applied with sterile gauze ("*Positive Control"*). On the contrary the rinse-only treatment by using a saline solution with phosphate buffer ("*Rinse-only Control"*) produced no decreases in *S. Aureus* with respect to the not treated sample, by suggesting that the simple irrigation of the wound, as it happens in many competition products, is not effective in preventing the biofilm recolonization.

Preliminary results showed that the non-woven fabric exemplified in Example 1 results to be particularly effective in the biofilm removal.

## Claims

1. **A non-woven** fabric for cleansing, debriding, reactivating and/or dressing wounds comprising or consisting of viscose fibers, polyethylene terephthalate fibers (PET) and polypropylene fibers (PP), wherein said viscose fibers are present in an amount comprised between 40 and 70%, said PET fibers are present in an amount comprised between 15 and 30%, and said polypropylene fibers are present in an amount comprised between 15 and 30% with respect to the total weight of the fabric.

2. The non-woven fabric according to claim 1, wherein said fibers are mechanically tied together so as to form multiple needle and ring structures.

3. The non-woven fabric according to claims 1 or 2, wherein said viscose fibers are present in an amount equal to 55%, said PET fibers are present in an amount equal to 25%, and said polypropylene fibers are present in an amount equal to 20% with respect to the total weight of the fabric.

4. The non-woven fabric according to any one of claims 1 to 3, wherein said viscose fibers, said polypropylene fibers and said polyethylene terephthalate fibers have a linear density comprised between 0.5-7.0 dtex and a length comprised between 10 and 120 mm, preferably wherein said viscose fibers and said polypropylene fibers have a linear density equal to 1.7 dtex and a length equal to 40 mm, and said polyethylene terephthalate fibers have a linear density equal to 3.3 dtex and a length equal to 60 mm.

5. The non-woven fabric according to any one of claims 1 to 4, having a weight comprised between 77 and 143 g/m², preferably equal to 110 g/m², and/or a thickness comprised between 1 and 1.62 mm.

6. The non-woven fabric according to any one of claims 1 to 5, having a percentage elongation at break in orthogonal direction (CD) comprised between 37-102%, and/or a tensile strength in orthogonal direction (CD) comprised between 50-128 N/5cm.

7. The non-woven fabric according to any one of claims 1 to 6, having an adsorption capacity comprised between 976-1496%, measured according to ISO 9073-6.

8. The non-woven fabric according to any one of the preceding claims, in form of glove or mitten.

9. The non-woven fabric according to claim 8, wherein said non-woven fabric is welded so as to form said glove or mitten by ultrasound technique.

10. The non-woven fabric according to any one of claims 1 to 9, soaked in an aqueous solution comprising an Aloe barbadensis extract, allantoin and/or poloxamer.

11. The non-woven fabric according to claim 10, wherein said aqueous solution further comprises linolealamidopropyl pg-dimonium chloride phosphate, disodium EDTA, sodium benzoate, polysorbate 20 and/or benzalkonium chloride.

12. The non-woven fabric according to any one of claims 1 to 11, wherein said non-woven fabric is sterile.

13. The non-woven fabric according to any one of claims 1 to 12, wherein said wounds are non-healing wounds.

14. A glove or mitten comprising or consisting of a non-woven fabric according to any one of claims 1 to 13.

15. A kit for cleansing, debriding, reactivating and/or dressing wounds comprising a non-woven fabric according to any one of claims 1 to 13 and/or a glove or mitten according to claim 14, and an aqueous solution for the treatment of wounds, preferably as defined in any one of claims 10 to 11.

## Patentansprüche

1. Vliesstoff zum Reinigen, Durchführen eines Debridements, Reaktivieren und/oder Verbandanlegen bei Wunden, umfassend oder bestehend aus Viskosefasern, Polyethylenterephthalatfasern (PET) und Polypropylenfasern (PP), wobei die Viskosefasern, in Bezug auf das Gesamtgewicht des Stoffs, in einer Menge, die zwischen 40 und 70 % umfasst, vorhanden sind, die PET-Fasern in einer Menge, die zwischen 15 und 30 % umfasst, vorhanden sind und die Polypropylenfasern in einer Menge, die zwischen 15 und 30 % umfasst, vorhanden sind.

2. Vliesstoff nach Anspruch 1, wobei die Fasern mechanisch miteinander verknüpft sind, um mehrere Nadel- und Ringstrukturen auszubilden.

3. Vliesstoff nach Anspruch 1 oder 2, wobei die Viskosefasern, in Bezug auf das Gesamtgewicht des Stoffs, in einer Menge gleich 55 % vorhanden sind, die PET-Fasern in einer Menge gleich 25 % vorhanden sind und die Polypropylenfasern in einer Menge gleich 20 % vorhanden sind.

4. Vliesstoff nach einem der Ansprüche 1 bis 3, wobei die Viskosefasern, die Polypropylenfasern und die Polyethylenterephthalatfasern eine lineare Dichte, die zwischen 0,5-7,0 dtex umfasst, und eine Länge, die zwischen 10 und 120 mm umfasst, aufweisen, wobei die Viskosefasern und die Polypropylenfasern vorzugsweise eine lineare Dichte gleich 1,7 dtex und eine Länge gleich 40 mm aufweisen, und die Polyethylenterephthalatfasern eine lineare Dichte gleich 3,3 dtex und eine Länge gleich 60 mm aufweisen.

5. Vliesstoff nach einem der Ansprüche 1 bis 4, der ein Gewicht, das zwischen 77 und 143 g/m² umfasst, vorzugsweise gleich 110 g/m² ist, und/oder eine Dicke, die zwischen 1 und 1,62 mm umfasst, aufweist.

6. Vliesstoff nach einem der Ansprüche 1 bis 5, der eine prozentuale Reißdehnung in orthogonaler Richtung (CD), die zwischen 37-102 % umfasst, und/oder eine Zugfestigkeit in orthogonaler Richtung (CD), die zwischen 50-128 N/5 cm umfasst, aufweist.

7. Vliesstoff nach einem der Ansprüche 1 bis 6, der eine Adsorptionskapazität, gemessen gemäß ISO 9073-6, aufweist, die zwischen 976-1496 % umfasst.

8. Vliesstoff nach einem der vorstehenden Ansprüche in Form eines Handschuhs oder Fausthandschuhs.

9. Vliesstoff nach Anspruch 8, wobei der Vliesstoff durch Ultraschalltechnik verschweißt wird, um den Handschuh oder Fausthandschuh auszubilden.

10. Vliesstoff nach einem der Ansprüche 1 bis 9, der in einer wässrigen Lösung, umfassend einen Extrakt von Aloe barbadensis, Allantoin und/oder Poloxamer, getränkt ist.

11. Vliesstoff nach Anspruch 10, wobei die wässrige Lösung ferner Linolealamidopropyl-PG-dimoniumchloridphosphat, Dinatrium-EDTA, Natriumbenzoat, Polysorbat 20 und/oder Benzalkoniumchlorid umfasst.

12. Vliesstoff nach einem der Ansprüche 1 bis 11, wobei der Vliesstoff steril ist.

13. Vliesstoff nach einem der Ansprüche 1 bis 12, wobei die Wunden nicht heilende Wunden sind.

14. Handschuh oder Fausthandschuh, umfassend oder bestehend aus einem Vliesstoff nach einem der Ansprüche 1 bis 13.

15. Kit zum Reinigen, Durchführen des Debridements, Reaktivieren und/oder Verbandanlegen bei Wunden, umfassend einen Vliesstoff nach einem der Ansprüche 1 bis 13 und/oder einen Handschuh oder Fausthandschuh nach Anspruch 14 und eine wässrige Lösung für die Behandlung von Wunden, vorzugsweise wie in einem der Ansprüche 10 bis 11 definiert.

## Revendications

1. Tissu non tissé pour le nettoyage, le débridement, la réactivation et/ou le pansement de plaies, comprenant des fibres de viscose, des fibres de polyéthylène téréphtalate (PET) et des fibres de polypropylène (PP) ou constitué de celles-ci, dans lequel lesdites fibres de viscose sont présentes en une quantité comprise entre 40 et 70 %, lesdites fibres de PET sont présentes en une quantité comprise entre 15 et 30 %, et lesdites fibres de polypropylène sont présentes en une quantité comprise entre 15 et 30 % par rapport au poids total du tissu.

2. Tissu non tissé selon la revendication 1, dans lequel lesdites fibres sont liées mécaniquement ensemble de manière à former multiples structures d'aiguilles et d'anneaux.

3. Tissu non tissé selon la revendication 1 ou 2, dans lequel lesdites fibres de viscose sont présentes en une quantité égale à 55 %, lesdites fibres de PET sont présentes en une quantité égale à 25 %, et lesdites fibres de polypropylène sont présentes en une quantité égale à 20 % par rapport au poids total du tissu.

4. Tissu non tissé selon l'une quelconque des revendications 1 à 3, dans lequel lesdites fibres de viscose, lesdites fibres de polypropylène et lesdites fibres de polyéthylène téréphtalate ont une densité linéaire comprise entre 0,5 et 7,0 dtex et une longueur comprise entre 10 et 120 mm, de préférence dans lequel lesdites fibres de viscose et lesdites fibres de polypropylène ont une densité linéaire égale à 1,7 dtex et une longueur égale à 40 mm, et lesdites fibres de polyéthylène téréphtalate ont une densité linéaire égale à 3,3 dtex et une longueur égale à 60 mm.

5. Tissu non tissé selon l'une quelconque des revendications 1 à 4, ayant un poids compris entre 77 et 143 g/m², de préférence égal à 110 g/m², et/ou une épaisseur comprise entre 1 et 1,62 mm.

6. Tissu non tissé selon l'une quelconque des revendications 1 à 5, ayant un pourcentage d'allongement à la rupture dans la direction orthogonale (CD) compris entre 37 et 102 %, et/ou une résistance à la traction dans la direction orthogonale (CD) comprise entre 50 et 128 N/5 cm.

7. Tissu non tissé selon l'une quelconque des revendications 1 à 6, ayant une capacité d'adsorption comprise entre 976 et 1496 %, mesurée selon la norme ISO 9073-6.

8. Tissu non tissé selon l'une quelconque des revendications précédentes, sous forme de gant ou de moufle.

9. Tissu non tissé selon la revendication 8, dans lequel ledit tissu non tissé est soudé de manière à former ledit gant ou ladite moufle par technique des ultrasons.

10. Tissu non tissé selon l'une quelconque des revendications 1 à 9, imbibé d'une solution aqueuse comprenant extrait d'Aloe *barbadensis,* allantoïne et/ou poloxamère.

11. Tissu non tissé selon la revendication 10, dans lequel ladite solution aqueuse comprend en outre phosphate de chlorure de linoléalamidopropyl-PG-dimonium, EDTA disodique, benzoate de sodium, polysorbate 20 et/ou chlorure de benzalkonium.

12. Tissu non tissé selon l'une quelconque des revendications 1 à 11, dans lequel ledit tissu non tissé est stérile.

13. Tissu non tissé selon l'une quelconque des revendications 1 à 12, dans lequel lesdites plaies sont des plaies non cicatrisantes.

14. Gant ou moufle comprenant un tissu non tissé selon l'une quelconque des revendications 1 à 13 ou constitué de celui-ci.

15. Kit pour le nettoyage, le débridement, la réactivation et/ou le pansement de plaies, comprenant un tissu non tissé selon l'une quelconque des revendications 1 à 13 et/ou un gant ou une moufle selon la revendication 14, et une solution aqueuse pour le traitement de plaies, de préférence telle que définie dans l'une quelconque des revendications 10 à 11.
